**Europäisches Patentamt**

**European Patent Office**

**Office européen des brevets**

(19)

(11) Veröffentlichungsnummer : **0 277 092 B1**

## (12) EUROPÄISCHE PATENTSCHRIFT

(45) Veröffentlichungstag der Patentschrift :
**29.01.92 Patentblatt 92/05**

(21) Anmeldenummer : **88810005.4**

(22) Anmeldetag : **08.01.88**

(51) Int. Cl.⁵ : **A61K 9/22,** A61M 31/00

(54) **Therapeutisches System für schwerlösliche Wirkstoffe.**

(30) Priorität : **14.01.87 CH 116/87**

(43) Veröffentlichungstag der Anmeldung :
**03.08.88 Patentblatt 88/31**

(45) Bekanntmachung des Hinweises auf die
Patenterteilung :
**29.01.92 Patentblatt 92/05**

(84) Benannte Vertragsstaaten :
**AT BE CH DE ES FR GB GR IT LI LU NL SE**

(56) Entgegenhaltungen :
**DE-A- 3 725 824**
**DE-A- 3 725 824**

(56) Entgegenhaltungen :
**FR-A- 2 327 764**
**GB-A- 2 173 704**
**JOURNAL OF MACROMOLECULAR SCIENCE REVIEWS ON MACROMOLECULAR CHEMISTRY AND PHYSICS, Band C23, Nr. 1, 1983, Seiten 61-126, Marcel Dekker, Inc., New York, US; R. LANGER et al.: "Chemical and physical structure of polymers as carriers for controlled release of bioactive agents: a review"**

(73) Patentinhaber : **CIBA-GEIGY AG**
**Klybeckstrasse 141**
**CH-4002 Basel (CH)**

(72) Erfinder : **Khanna, Satish Chandra, Dr.**
**Rütistrasse 26**
**CH-4103 Bottmingen (CH)**

**Beschreibung**

Gegenstand der vorliegenden Erfindung ist ein oral zu applizierendes und systemisch wirkendes therapeutisches System mit einer wirkstoffhaltigen Kammer, welches in dieser Kammer schwerlösliche Wirkstoffe, Quellmittel und gegebenenfalls wasserlösliche Stoffe zur Erzeugung eines osmotischen Drucks enthält, sowie Verfahren zur Herstellung eines solchen Systems.

Therapeutische Systeme zur Erzielung einer systemischen Wirkung sind bekannt. Beim peroralen System OROS®: Oral Osmotic System (Alza Corp.), welches von F. Theeuwes in J. Pharm. Sc. Vol. 64, 12, 1987-1991 (1975) beschrieben ist und in seiner einfachsten Version die Form einer konventionellen, beschichteten Tablette besitzt, dringen wässrige Körperflüssigkeiten kontinuierlich durch die als semipermeable Membran wirkende äussere Schicht in das System ein, erreichen den im Kern in fester Form vorliegenden Wirkstoff und lösen diesen auf. Bei ausreichender Wasserlöslichkeit wird die wirkstoffhaltige Lösung durch den sich aufbauenden osmotischen Druck mit konstanter Geschwindigkeit durch eine Austrittsöffnung von ca. 100 - 250 Mikrometer Weite abgegeben.

Diese Darreichungsform erzielt eine ausreichende Abgabemenge des Wirkstoffes und damit den gewünschten therapeutischen Effekt, wenn der im Kern vorhandene Wirkstoff einen genügend hohen eigenen osmotischen Druck erzeugen kann. Voraussetzung dafür ist ein genügend hoher Gehalt an wasserlöslichem Wirkstoff mit entsprechend niedrigem Hilfsstoffanteil im Kern.

Daher sind die OROS®-Systeme für schwerlösliche Wirkstoffe ungeeignet. Besonders bei hoch dosierbaren Wirkstoffen wie Carbamazepin wäre oder osmotische Druck zu gering. Zur Lösung dieses Problems werden in der U.S. Patentschrift 4,111,202 Doppelkammersysteme für schwerlösliche Wirkstoffe ("Push-Pull"-Systeme) beschrieben, welche in einer Kammer den Wirkstoff bzw. die Wirkstofformulierung und in einer darunter liegenden Kammer wasserlösliche Verbindungen zur Erzeugung eines osmotischen Drucks, z.B. Salze oder Zucker, enthalten. Die beiden Kammern sind voneinander durch eine flexible Wand getrennt und nach aussen durch eine starre, aber wasserdurchlässige semipermeable Membran abgeschlossen. Bei Zutritt von Wasser bewirkt der sich aufbauende osmotische Druck eine Volumenvergrösserung der unteren Kammer. Da die semipermeable Wand starr ist, wirkt der osmotische Druch auf die sich dabei ausdehnende flexible Trennwand und drückt den Inhalt der wirkstoffhaltigen Kammer aus dem System.

Die Herstellung von "Push-Pull"-Systemen ist technisch aufwendig, da in solche Darreichungsformen eine flexible Trennwand eingebaut werden muss, die aus einem anderen Material als die semipermeable Hülle besteht. Ausserdem lassen sich für schwerlösliche und hoch zu dosierende Wirkstoffe wie z.B. Carbamazepin mit z.B. mehr als 200 mg Dosierung nur voluminöse "Push-Pull"-Systeme herstellen, deren Einnahme problematisch sein kann.

In der Europäischen Patentanmeldung 52917 werden Doppelkammersysteme für schwerlösliche Wirkstoffe ohne Trennwand beschrieben. Das osmotische Treibmittel ist in der wirkstoffhaltigen Kammer enthalten. Die darunterliegende Kammer besteht aus quellfähigen Polymeren wie Polyvinylpyrrolidon. Der sich aufbauende osmotische Druck bewirkt vermehrte Flüssigkeitsaufnahme des Systems, wodurch die Quellung beschleunigt wird. Der Quelldruck erzeugt eine Volumenvergrösserung nur jener Kammer, welche aus quellfähigen Polymeren besteht und drückt, da die semipermeable Wand starr ist, den Inhalt der wirkstoffhaltigen Kammer durch eine Oeffnung nach aussen.

Die in der Europäischen Patentanmeldung 52917 beschriebene Darreichungsform ist als Zweischichttablette mit Ummantelung bzw. Beschichtung aufzufassen. Deren Herstellung ist gegenüber einfachen Manteltabletten bzw. beschichteten Tabletten technisch aufwendig. So muss der Kompressionsvorgang in mindestens zwei Stufen durchgeführt werden. Bei der üblichen Kompression von verschiedenen Granulaten werden hohe Anforderungen an die einheitliche Körnung der Granulatkomponenten, welche miteinander verpresst werden, gestellt. Auf die Beschreibung der Herstellung von Mehrschichttabletten, deren technischen Probleme und Anforderungen an die verwendeten Granulate in Hagers Handbuch der Pharmazeutischen Praxis, Springer Verlag 1971, im folgenden "Hager", Band VIIa, S. 710 unten und S. 723 unten bis S. 725 wird verwiesen.

Der vorliegenden Erfindung liegt die Aufgabe zugrunde, orale therapeutische Systeme für schwerlösliche Wirkstoffe mit nur einer Kammer herzustellen, deren Grösse so klein zu halten ist, dass sie den bisher bekannten oralen osmotischen Systemen für lösliche Wirkstoffe entspricht.

Diese Aufgabe wird durch die Wahl eines vorteilhaften Quellmittels gelöst, das sich zur Herstellung von Einkammersystemen eignet und eine ausreichende Abgabeleistung des Einkammersystems bewirkt. Bekannte Quellmittel wie Polyvinylpyrrolidon, Polyethylenoxid, Polymethacrylat etc. sind in Einkammersystemen nachteilig, da der Quelldruck so gross ist, dass im Kontakt mit Wasser die semipermeable Wand aufgesprengt wird und das ganze System bereits im Magen nach kurzer Zeit zerfällt.

Die Erfindung betrifft ein peroral zu applizierendes und systemisch wirkendes therapeutisches System in Form eines beschichteten oder ummantelten Einkammersystems für die Darreichung von in Wasser schwerlöslichen Wirkstoffen ausgenommen Carbamapezin. Therapeutische Systeme für Carbamazepin sind in der Deutschen Offenlegungsschrift DE-A-3 725 824 mit Priorität vom 7.8.1986 offenbart, welche am 11.2.1988 publiziert wurde.

Das erfindungsgemässe therapeutische System besteht aus:

a) einer Hülle aus einem für Wasser durchlässigen und für die Komponenten des wirkstoffhaltigen Kerns undurchlässigen Material,

b) einem Kern enthaltend den in Wasser schwerlöslichen Wirkstoff oder eine Wirkstoffkombination, ein hydrophiles, polymeres Quellmittel bestehend aus einer Mischung des Vinylpyrrolidon-Vinylacetat-Copolymerisats mit Ethylenoxidhomopolymerisat, gegebenenfalls einen wasserlöslichen Stoff zur Induzierung der Osmose und gegebenenfalls weitere pharmazeutisch annehmbare Hilfsstoffe und

c) einer Passage durch die Hülle a) für den Transport der im Kern enthaltenen Bestandteile in die umgebende wässrige Körperflüssigkeit.

Die weiter vorn und im folgenden verwendeten Definitionen und Begriffe haben im Rahmen der Beschreibung der vorliegenden Erfindung vorzugsweise die folgenden Bedeutungen:

Die Hülle a) aus einem für Wasser durchlässigen und für die Komponenten des wirkstoffhaltigen Kerns undurchlässigen Material kann als semipermeable Membran aufgefasst werden, welche zwar für Wasser durchlässig, für die im Kern der Darreichungsform enthaltenen Bestandteile wie Wirkstoffe, Quellmittel, Hilfsstoffe etc. aber undurchlässig ist.

Zur Herstellung der Hülle aus semipermeablem Material eignen sich die in der Literatur, z.B. in der U.S. Patentschrift Nr. 3 916 899 und Nr. 3 977 404, beschriebenen polymeren Stoffe, die im Gastrointestinaltrakt nicht metabolisiert werden, d.h. unverändert ausgeschieden werden. Beispielsweise können acylierte Cellulose-derivate (Celluloseester), die durch Acetylgruppen ein- bis dreifach oder durch Acetylgruppen ein- bis zweifach und einen weiteren von Acetyl verschiedenen Acylrest substituiert sind, verwendet werden, z.B. Celluloseacetat, Cellulosetriacetat, Celluloseacetat-ethylcarbamat, Celluloseacetatphthalat, Celluloseacetat-methylcarbamat, Celluloseacetat-succinat, Celluloseacetat-dimethylaminoacetat, Celluloseacetat-ethylcarbonat, Celluloseacetat-chloracetat, Celluloseacetat-ethyloxalat, Celluloseacetat-methylsulfonat, Celluloseacetat-butylsulfonat, Celluloseacetat-propionat, Celluloseacetat-diethylaminoacetat, Celluloseacetat-octat, Celluloseacetat-laurat, Celluloseacetat-p-toluolsulfonat, Celluloseacetat-butyrat und andere Celluloseacetatderivate sowie Agaracetat und Amylose acetat. Als semipermeables Membranmaterial eignen sich auch Hydroxypropylmethylcellulose und polymere Epoxide, Copolymere aus Alkylenoxiden und Alkylglycidylethern, Polyglycole oder Polymilchsäurederivate und weitere Abkömmlinge davon. Ferner können auch Mischungen, wie z.B. von an sich wasserunlöslichen Acrylaten (z.B. Copolymerisat von Acrylsäureethylester und Methacrylsäuremethylester) verwendet werden.

Der im Kern des erfindungsgemässen therapeutischen Systems enthaltene Wirkstoff ist in Wasser, insbesondere in Körperflüssigkeiten wie Magen- oder Darmsäften, schwerlöslich und daher nur schlecht resorbierbar. Bei Verwendung von konventionellen peroralen Darreichungsformen wie Dragées oder Tabletten ist meistens eine hohe Dosierung erforderlich, welche bei der stossweisen Abgabe im Magen die Schleimhäute entsprechend belastet. So ist z.B. in den kommerziell erhältlichen Präparaten mit Acetylsalicylsäure in einer Tablette bis zu 500 mg Wirkstoff enthalten.

Geeignete Wirkstoffe für das erfindungsgemässe therapeutische System haben vorzugsweise eine Löslichkeit von weniger als 5 g in 100 ml Wasser bei 37°C und sind aus verschiedenen Indikationsgebieten ausgewählt, z.B. aus der Gruppe der Hypnotika/Sedativa, Psychopharmaka, Antihypertensiva, Muskelrelaxantia, Analgetika und/oder Antiinflammatorika, Spasmolytika, Gastritis- und/oder Ulkusmittel, Antikonvulsiva, Diuretika etc.

Für die in der Beschreibung der Erfindung bezeichneten Wirkstoffe werden, wenn nicht anders angegeben, die von der Weltgesundheitsorganisation (WHO) vorgeschlagenen Freinamen (Recommended International Non-proprietary Names - INN Namen) verwendet, welche der jährlich neu erscheinenden "Roten Liste" (Herausgeber: Bundesverband der Deutschen Pharmazeutischen Industrie, D-6000 Frankfurt a.M.) sowie dem Merck-Index (Tenth Edition) entnommen sind.

Besonders geeignete Wirkstoffe sind insbesondere Hypnotika/Sedativa wie Azacyclonol oder Buclizin, Psychopharmaka wie Diazepam, Chlordiazepoxid, Oxazepam, Oxanamid, Hydroxyphenamat, Phenaglycodol, Haloperidol, Perphenazin, Thiotixen etc., Antihypertensiva wie Mebutamat, Antiepileptika wie Diphenylhydantoin, Metharbital, Methsuximid, Paramethadion, Phensuximid, Primidon, Trimethadion oder Carbamazepin, Analgetika und/oder Antiinflammatorika, z.B. Acetaminophen, Actylsalicylsäure, etc., ZNS-Stimulanzien, z.B. Bronchiospasmolytika wie Theophyllin, Calciumantagonisten wie Nifedipin oder Nicardipin, antibakterielle Mittel wir Nitrofurantoin, Iodoxuridin, Pencilline wie Ampicillin, Cephalosporine wie Cefaclor, Cefalexin, Cefsulodin oder Cefotiam u.a.

Es können ebenfalls die üblichen Kombinationen der genannten Wirkstoffe und ihrer Analoga, welche hier nicht genannt sind, im erfindungsgemässen therapeutischen System enthalten sein.

Für das erfindungsgemässen therapeutische System werden die Wirkstoffe in feinkörniger Form verwendet. Der Begriff "feinkörnige Form" umfasst mikronisierte, amorphe wasserfreie und kristalline wasserfreie Formen und kristalline Hydratformen. Bevorzugt sind mikronisierte kristalline, wasserfreie Formen. Die Teilchengrösse muss so klein sein, dass eine staufreie Abgabe des Wirkstoffs durch die Oeffnung in der semipermeable Membran gewährleistet ist, welche einen bevorzugten Durchmesser von ca. 0,4 - 0,8 mm hat. Ausserdem erfolgt bei kleiner Teichengrösse eine verbesserte Resorption von dispergierten Teilchen der schwerlöslichen Wirkstoffe. In einer bevorzugten Ausführungsform werden wasserfreie Kristalle von Wirkstoffen mit einer Teilchengrösse kleiner als 100 µm, insbesondere kleiner als 20 µm, verwendet. Dosisangaben können der jeweils gültigen "Roten Liste" oder dem PDR (Physicians' Desk Reference, Medical Economics Co.) entnommen werden.

Das hydrophile Quellmittel, welches im Kern b) enthalten ist, ist ein Polymer, das mit Wasser aus der im Gastrointestinaltrakt enthaltenen wässrigen Körperflüssigkeit in Wechselwirkung tritt, quillt und sich bis zu einem Gleichgewichtszustand ausdehnen kann. Das Quellmittel besitzt die Fähigkeit, grosse Mengen Wasser zu absorbieren und den zur Funktion des therapeutischen Systems notwendigen Quelldruck zu erzeugen.

Das im erfindungsgemässen therapeutischen System verwendete hydrophile, polymere Quellmittel besteht aus einer Mischung des Vinylpyrrolidon-Vinylacetat-Copolymerisats mit Ethylenoxidhomopolymerisat. Diese Mischung hat den überraschenden Vorteil, dass der bei der Quellung erzeugte Quelldruck nicht zu einer Aufsprengung des Systems führt und die Quellungsgeschwindigkeit gleichförmig ist, so dass annähernd konstante Wirkstoffmengen vom System abgegeben werden. Die Vinylpyrrolidon-Vinylacetat-Copolymerisat-Komponente hat vorzugsweise ein Molekulargewicht von 60 000 ± 15 000. Bevorzugt ist das den Copolymeren zugrunde liegende Monomerenverhältnis Vinylpyrrolidon zu Vinylacetat von ca. 60 : 40 (Gew.%). Das Vinylpyrrolidon-Vinylacetat-Copolymerisat hat folgende Eigenschaften:

Reinheit: 95 % (Rest Wasser), unlöslich in Ether, aliphatischen Kohlenwasserstoffen, sehr gut löslich in Wasser, Ethyl- und Isopropylalkohol, Methylenchlorid, Glycerin und 1,2-Propylenglycol, pH-Wert einer 10 %igen wässrigen Lösung 3 - 5, Viskosität (10 %ige wässrige Lösung): 5 mPas, siehe H.P. Fiedler, Lexikon der Hilfsstoffe, H.P. Fiedler, Editio Cantor 1982.

Vinylpyrrolidon-Vinylacetat-Copolymerisate sind bekannt und/oder auf an sich bekannte Weise in beliebigem Mischungsverhältnis der Monomeren herstellbar. Das bevorzugte 60:40-Copolymerisat ist z.b. kommerziell unter der Bezeichung Kollidon® VA 64 (BASF) erhältlich.

Das Vinylpyrrolidon-Vinylacetat-Copolymerisat ist mit Ethylenoxidhomopolymeren vermischt, die einen Polymerisationsgrad von ca. $2,0 \times 10^3$ - $1,0 \times 10^5$ und ein entsprechendes ungefähres Molekulargewicht von ca. $1,0 \times 10^5$ - $5,0 \times 10^6$ und folgende Eigenschaften haben:

Ab Molgewicht etwa 4000 feste, wachsartige Substanzen. In jedem Verhältnis mit Wasser und untereinander mischbar. Löslich in Methanol, Aethanol, Aceton, Methylenchlorid.

Ethylenoxidhomopolymerisate (Polyethylenglycole) sind bekannt und für verschiedene Polymerisationsgrade kommerziell erhältlich, z.B. unter der Bezeichnung Polyox® (Union Carbide). Bevorzugt wird Polyox® (Koagulans) mit einem Molgewicht grösser als $1,0 \times 10^6$ verwendet.

In einer bevorzugten Ausführungsform der Erfindung wird ein 1:1-Gemisch (Gew.) von Vinylpyrrolidon-Vinylacetat-Copolymerisat (Handelsform: Kollidon® VA 64) mit Ethylenoxidhomopolymerisat (Handelsform Polyox® MG: $5 \times 10^6$) verwendet.

Das hydrophile, polymere Quellmittel kann in Gewichtsteilen von ca. 5 - 60 %, bezogen auf das Gesamtgewicht des betreffenden therapeutischen Systems, im Kern vorhanden sein.

Die im Kern zusätzlich zu den Quellmitteln gegebenenfalls enthaltenen wasserlöslichen Stoffe zur Induzierung der Osmose erzeugen durch das herrschende Konzentrationsgefälle einen osmotischen Druck nach Eindringen von Wasser durch die semipermeable Hülle und verstärken den Quelldruck. Da die semipermeable Hüllse a) starr oder zumindest nur wenig elastisch ist, kann der durch Osmose und Quellung erzeugte Druck nur durch Abgabe von im Kern enthaltenem Material durch die in der Membran befindliche Passage c) ausgeglichen werden.

Als wasserlösliche Stoffe zur Induzierung der Osmose sind im Prinzip alle wassserlöslichen Stoffe geeignet, deren Verwendung in der Pharmazie unbedenklich ist, z.B. die in Pharmakopöen oder in "Hager" sowie in Remington's Pharmaceutical Science erwähnten wasserlöslichen Hilfsstoffe. Insbesondere sind pharmazeutisch verwendbare, wasserlösliche Salze von anorganischen oder organischen Säure oder nichtionische organische Stoffe mit besonders grosser Wasserlöslichkeit, z.B. Kohlehydrate wie Zucker, oder Aminosäuren, z.B. Glycin, geeignet.

Solche wasserlöslichen Stoffe zur Induzierung der Osmose sind beispielsweise anorganische Salze wie Magnesiumchlorid oder -sulfat, Lithium-, Natrium- oder Kaliumchlorid, Lithium-, Natrium- oder Kaliumsulfat oder Natrium- oder Kaliumhydrogen- oder -dihydrogenphosphat, Salze von organischen Säuren wie Natrium- oder Kaliumacetat, Magnesiumsuccinat, Natriumbenzoat, Natriumcitrat oder Natriumascorbat, Kohlehydrate wie Arabinose, Ribose oder Xylose (Pentosen), Glucose, Fructose, Galactose oder Mannose (Hexosen), Sucrose, Maltose oder Lactose (Disaccharide) oder Raffinose (Trisaccharide), wasserlösliche Aminosäuren wie Glycin, Leucin, Alanin oder Methionin, Harnstoff etc. sowie Gemische davon. Diese wasserlöslichen Hilfsstoffe können in Gewichtsanteilen von ca. 0,01 - 35 %, bezogen auf das Gesamtgewicht des betreffenden therapeutischen Systems, im Kern vorhanden sein.

Der Kern b) kann zusätzlich zu den wasserlöslichen Stoffen zur Induzierung der Osmose und dem hydrophilen, polymeren Quellmittel weitere pharmazeutische annehmbare Hilfsstoffe enthalten. Solche Hilfsstoffe sind beispielsweise Schutzkolloide, welche ein eventuelles Kristallwachstum von Wirkstoffen in Wasser inhibieren und die Bildung von grösseren Hydratkristallen aus feinkörnigen Teilchen verhindern. Vor allem können Schutzkolloide die Bildung grösserer Hydratkristalle aus wasserfreien Modifikationen oder amorphen Teilchen verhindern. Wie weiter vorn erwähnt, ist die Bildung von grösseren Kristallen für die kontinuierlich Abgabeleistung des therapeutischen Systems nachteilig (Verstopfen der Passage, durch welche der Transport des Wirkstoffs aus dem System erfolgt und Verhinderung der Abgabe des Wirkstoffs).

Geeignete Schutzkolloide sind insbesondere methylierte Cellulosederivate, z.B. Methylcellulose mit einem Methoxygehalt von ca. 27,0 bis 32,0 % und einem Substitutionsgrad von ca. 1,75 bis 2,1, oder Methylhydroxypropylcellulose mit einem Gehalt von ca. 16,0 - 30,0 % Methoxy- und 4,0 - 32,0 % Hydroxypropoxy-Gruppen. Schutzkolloide wie Methylhydroxypropylcellulose können in dem erfindungsgemässen therapeutischen System in einem bevorzugten Mengenverhältnis von ca. 0,5 - 10 %, bezogen auf die Wirkstoffmenge, enthalten sein.

So wird beispielsweise durch Zusatz dieses Schutzkolloids das in wässriger Phase beobachtbare Wachstum von wasserfreien Carbamazepin-Mikrokristallen mit einer Grösse von bis zu 100 μm, insbesondere 1 - 20 μm, oder Hydraten davon mit ähnlicher Grösse zu nadelförmigen Hydraten mit einer Grösse von ca. 100 - 500 μm verhindert bzw. verlangsamt.

Vom erfindungsgemässen therapeutischen System können Mikrokristalle mit einer durchschnittlichen Grösse von ca. 1 - 20 μm an den Gastrointestinaltrakt abgegeben werden. Wirkstoffe können also in besonders fein dispergierter Form schneller gelöst werden.

Weitere Hilfsstoffe sind z.B. Weichmacher, welche die Fliesseigenschaften und die Handhabung des hydrophilen, polymeren Materials während der Herstellung des Kerns verbessern, z.B. Glycerin, Triethylcitrat, Diethylphthalat, Diethylsebacat u.ä. Die Menge an zugesetztem Weichmacher beträgt ca. 0,01 bis 20 Gew.-% bezogen auf die Gesamtmenge des therapeutischen Systems.

Es können auch grenzflächenaktive Stoffe, sog. Tenside, bei der Herstellung des Kerns zugesetzt werden, z.B. anionischen Tenside vom Typ Alkylsulfat, z.B. Natrium-, Kalium- oder Magnesium-n-dodecylsulfat, -n-tetradecyl-sulfat, -n-hexadecyl-sulfat oder -n-octa-decyl-sulfat, Alkylethersulfat, z.B. Natrium-, Kalium- oder Magnesium-n-dodecyloxyethylsulfat, -n-tetradecyloxyethylsulfat, -n-hexadecyl-oxyethylsulfat oder -n-octadecyloxyethylsulfat oder Alkansulfonat, z.B. Natrium-, Kalium- oder Magnesium-n-dodecansulonat, -n-tetradecansulfonat, -n-hexadecansulfonat oder -n-octadecansulfonat.

Geeignete Tenside sind ferner nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere von Typ Pluronics® (BWC) oder Synperonic® (ICI).

Weitere Hilfsstoffe sind z.B. pulverförmige Trägermaterialien wie Lactose, Saccharose, Sorbit, Mannit, Stärke, z.B. Kartoffelstärke, Maisstärke oder Amylopektin, oder Cellulose, insbesondere mikrokristalline Cellulose.

Der Begriff "Passage durch die Hülle a) für den Transport der im Kern enthaltenen Bestandteile in die umgebende wässrige Körperflüssigkeit" umfasst Vorrichtungen sowie Methoden, die geeignet sind, die Wirkstoffzubereitung aus dem Kern des therapeutischen Systems freizusetzen. Der Begriff umfasst Durchgänge, Oeffnungen, Bohrungen, Löcher u.ä. durch die als semipermeable Membran wirkende Hülle a), welche zwischen der Oberfläche der Hülle und dem Kern eine Verbindung herstellen. Die Passage kann durch mechanisches Bohren oder Laserbohren oder durch Zersetzen eines abbaubaren Bestandteils, z.B. eines Gelatinestopfens, unter Bildung einer Passage in der Hülle des therapeutischen Systems hergestellt werden. Bei einer Ausführungsform kann sich die Passage als Reaktion auf den hydrostatischen Druck bilden, welcher auf das therapeutischen System einwirkt. Bei einer anderen Ausführungsform können zwei oder mehrere Durchgänge hergestellt werden, die sich an einer beliebigen Stelle des Systems befinden. Die Passage kann auch durch mechanisches Aufbrechen der Schichten während der Anwendung des Systems entstehen. Die Passage hat einen minimalen Durchmesser, der von der Korngrösse der Wirkstoffkristalle abhängig ist. Der Durchmesser der Passage muss grösser als die durchschnittliche Länge der Wirkstoffkristalle sein. Der maximale Durchmesser ist ebenfalls annähernd festgelegt. Dieser darf nur so gross sein, dass das Eindringen von wässriger Körperflüssigkeit durch Konvektion in das therapeutische System vermieden wird. Eine genaue Beschreibung der Herstellung der Passage und der maximalen und minimalen Dimensionen davon ist in den U.S. Patentschriften 3 485 770 und 3 916 899 und den dazugehörigen Zeichnungen enthalten.

Das erfindungsgemässe therapeutische System kann unterschiedlich geformt sein und beispielsweise rund, oval, oblong, zylindrisch u.ä. sein sowie verschiedene Grössen in Abhängigkeit von der Füllmenge haben. Das therapeutische System kann ausserdem transparent, farblos oder gefärbt und gegebenenfalls beschriftet sein, um diesem Produkt ein individuelles Aussehen bzw. sofortige Erkennbarkeit zu verleihen.

Das erfindungsgemässe therapeutische System besitzt wertvolle pharmakologische Eigenschaften und kann bei der peroralen Applikation von schwerlöslichen Wirkstoffen verwendet werden. Dabei wird gegenüber den bisher üblichen festen Darreichungsformen wie Tabletten und Dragées ein verbesserter therapeutischer Effekt erreicht.

Die vorliegende Erfindung betrifft insbesondere ein therapeutisches System bestehend aus

a) einer Hülle aus für Wasser durchlässiger und für die Komponenten des wirkstoffhaltigen Kerns sowie für die in Magen- oder Darmsaft enthaltenen Ionen undurchlässiger, acylierter Cellulose,

b) einem Kern enthalten den in Wasser schwerlöslichen Wirkstoff ausgenommen Carbamazepin oder eine Wirkstoffkombination, das 1:1-Gemisch (Gew.) von Vinylpyrrolidon-Vinylacetat-Copolymerisat mit Ethylenoxidhomopolymerisat, Natrium- oder Kaliumchlorid zur Induzierung der Osmose sowie gegebenenfalls weitere pharmazeutisch annehmbare Hilfsstoffe und

c) einer Passage durch die Hülle a) für den Transport der im Kern enthaltenen Bestandteile in die umgebende wässrige Körperflüssigkeit.

Die Erfindung betrifft in erster Linie ein therapeutisches System bestehend aus

a) einer Hülle aus für Wasser durchlässiger und für die Komponenten des wirkstoffhaltigen Kerns sowie für die in Magen- oder Darmsaft enthaltenen Ionen undurchlässigem Celluloseacetat,

b) einem Kern enthaltend den in Wasser schwerlöslichen Wirkstoff ausgenommen Carbamazepin oder eine Wirkstoffkombination, das 1:1-Gemisch (Gew.) aus Vinylpyrrolidon-Vinylacetat-Copolymerisat mit einem Molekulargewicht von 60000 ± 15000 und einem Monomerenverhältnis von ca. 60:40 (Gew.-%) und Ethylenoxidhomopolymerisat mit einem Polymerisationsgrad von 2000 bis 100000 und

c) einer Passage durch die Hülle a) für den Transport der im Kern enthaltenen Bestandteile in die umgebende wässrige Körperflüssigkeit.

Die vorliegende Erfindung betrifft vor allem ein therapeutisches System für die perorale Applikation von schwerlöslichen Wirkstoffen mit den in den Beispielen angegebenen Zusammensetzungen.

Das erfindungsgemässe therapeutische System wird nach an sich bekannten Verfahren hergestellt, z.B. indem man die Bestandteile des Kerns zerkleinert, miteinander vermischt, granuliert und zusammenpresst, den Kern mit einer Hülle überzieht und gegebenenfalls die Passage durch die Hülle a) für den Transport der im Kern enthaltenen Bestandteile, z.B. eine Oeffnung durch die semipermeable Membran, anbringt. Bei Wirkstoffen, welche Hydrate bilden, werden die Hydratkristalle oder nach Möglichkeit eine wasserfreie Kristallform bis hin zu einer mittleren Teilchengrösse von ca. 5 µm zerkleinert. Diese Teilchen, insbesondere Mikrokristalle, werden mit den den Kern der Darreichungsform bildenden Bestandteilen vermischt und granuliert, z.B. indem man die Hilfsstoffe wie z.B. Schutzkolloide wie Methylhydroxypropylcellulose oder Methylcellulose, Natriumchlorid sowie Polyox® mit dem Wirkstoff vermischt und diese Mischung mit einer Lösung Polyvinylpyrrolidon/Polyvinylacetat in einem organischen Lösungsmittel oder Wasser versetzt, das Lösungsmittel abzieht, den Rückstand granuliert und trocknet. Das Granulat wird anschliessend gepresst und zu Formlingen, z.B. Tablettenkernen, gegebenenfalls unter Zusatz von Gleitmitteln, z.B. Magnesiumstearat, konventioneller Form und Grösse von z.B. ca. 5-12 mm Durchmesser (runde Formen) und ca. 4-8 mm (breit) sowie ca. 10-22 mm (längs-oblonge Formen) ausgestanzt.

Geeignet sind alle Lösungsmittel, worin Copolymere von Polyvinylpyrrolidon und Polyvinylacetat löslich sind, insbesondere Wasser oder Niederalkanole wie Methanol, Aethanol oder Isopropanol.

Die semipermeable Hülle kann auf den den Wirkstoff enthaltenden Kern durch Aufgiessen, Formen, Sprühen oder Eintauchen der Kapsel in das die semipermeable Hülle bildende Material aufgebracht werden. Ein anderes Verfahren, das zum Aufbringen der Hülle angewandt werden kann, besteht im Luftverwirbeln (air suspension procedure). Dieses Verfahren besteht darin, dass man die Massen (Kapseln bzw. Kapselkerne) in einem Luftstrom und einem die Hülle bildenden Mittel suspendiert und stürzt, bis die Hülle den Kern umgibt und überzieht. Das Luftverwirbelungs-Verfahren ist in der U.S. Patentschrift 2 799 241 sowie im J.Am. Pharm. Assoc., Bd. 48, S. 451 - 459, 1979, und im Band 49, S. 82 bis 84, 1980, beschrieben. Andere bevorzugte Standardverfahren sind beispielsweise das Kessellackierungsverfahren (spray pan), welches in Remington's Pharmaceutical Sciences, 14th Edition, auf den Seiten 1686 - 1687 beschrieben ist.

Die Passage in der semipermeablen Hülle kann anschliessend durch Bohren mechanisch oder mit Laser erzeugt werden. Die folgenden Beispiele illustrieren die Erfindung.

Beispiel 1:

Therapeutisches System für Acetylsalicylsäure

```
Kern
Acetylsalicylsäure                                    500 mg
Mikrokristalline Cellulose (Avicel®)                   40 mg
Vinylpyrrolidon/Vinylacetat -60:40- Copolmyeres
(Kollidon® VA 64])                                    160 mg
Polyethylenglycol (MG:5x10⁶ -Polyox®-Coagulant)        80 mg
Natriumchlorid                                        120 mg
Magnesiumstearat                                       20 mg
                                                   = 1000 mg


Hülle
Celluloseacetat 320                                    20 mg
Celluloseacetat 39.8                                   30 mg
Polyethylenglycol 4000                                  6 mg
                                               =       56 mg
                                               ─────────────

                    Gesamtgewicht         ca.  1056 mg
                                          ════════════════
```

Man vermischt getrocknete Acetylsalicylsäure, welche bis zu einer durchschnittlichen Korngrösse von 5-10 μm trocken vermahlen wird und Natriumchlorid in einem Planetenmischer. Diese Mischung wird mit einem Teil des Vinylpyrrolidon/Vinylacetat-Copolymeren, gelöst in einer Methanol/Isopropanol-Mischung, granuliert. Das Gemisch wird durch ein Sieb gedrückt, und die erhältlichen Granulate werden im Vakuum getrocknet.

Das trockene Granulat wird mit der restlichen Menge Vinylpyrrolidon/Vinylacetat-Copolymeren, Avicel® und Magnesiumstearat vermischt. Das homogene Gemisch wird anschliessend komprimiert und ausgestanzt (Stempelgrösse 21 x 9 mm).

Die erhältlichen Tablettenkerne werden in einem Fliessbettbeschichter (Aeromatic Strea®1) mit einem organischen Lack enthaltend die Bestandteile der Hülle des therapeutischen Systems beschichtet. Die beschichteten Tabletten werden in einem Trockenschrank bei 40°C 48 Stunden lang getrocknet. Eine Oeffnung von 750 μm Durchmesser wird auf jeder Tablette mit einem Bohrer oder mit Laser angebracht.

Beispiel 2:

Therapeutisches System für Theophyllin

<u>Kern</u>

| | |
|---|---|
| Theophyllin | 200 mg |
| Mikrokristalline Cellulose (Avicel®) | 20 mg |
| Kollidon® VA 64 | 80 mg |
| Polyethylenglycol (MG:5x10$^6$-Polyox®-Coagulant) | 80 mg |
| Magnesiumstearat | 10 mg |
| | = 500 mg |

<u>Hülle</u>

| | |
|---|---|
| Celluloseacetat 320 | 23 mg |
| Celluloseacetat 39.8 | 10 mg |
| Polyethylenglycol 4000 | 4 mg |
| | = 37 mg |

Gesamtgewicht ca. 537 mg

Die Herstellung der Darreichungsform erfolgt analog Beispiel 1 (Stempelgrösse 10,5 mm Durchmesser), indem man Theophyllin (getrocknet) und zu einer durchschnittlichen Korngrösse von ca. 5,0 bis 10,0 µm trocken vermahlen verwendet.

Beispiel 3:

Therapeutisches System für Nifedipin

<u>Kern</u>

| | |
|---|---|
| Nifedipin | 50,0 mg |
| Kollidon® VA 64 | 18,0 mg |
| Polyethylenglycol (MG:5x10$^6$-Polyox®-Coagulant) | 20,0 mg |
| Natriumchlorid | 20,0 mg |
| Magnesiumstearat | 2,0 mg |
| | = 110,0 mg |

<u>Hülle</u>

| | |
|---|---|
| Celluloseacetat 320 | 15,0 mg |
| Polyethylenglycol 4000 | 2,0 mg |
| | = 17,0 mg |

Gesamtgewicht ca. 127,0 mg

Die Herstellung der Darreichungsform erfolgt analog Beispiel 1 (Stempelgrösse 7 mm Durchmesser), indem man Nifedipin (getrocknet) und zu einer durchschnittlichen Korngrösse von ca. 5,0 bis 10,0 μm trocken vermahlen verwendet.

**Patentansprüche**

**Patentansprüche Für folgenden Vertragsstaaten : AT, BE, CH, DE, FR, GB, IT, LI, LU, NL, SE**

1. Perorales therapeutisches System für die Darreichung von in Wasser schwerlöslichen Wirkstoffen ausgenommen Carbamazepin bestehend aus
a) einer Hülle aus einem für Wasser durchlässigen und für die Komponenten des wirkstoffhaltigen Kerns undurchlässigen Material,
b) einem Kern enthaltend den in Wasser schwerlöslichen Wirkstoff oder eine Wirkstoffkombination, ein hydrophiles, polymeres Quellmittel bestehend aus einer Mischung des Vinylpyrrolidon-Vinylacetat-Copolymerisats mit Ethylenoxidhomopolymerisat, gegebenenfalls einen wasserlöslichen Stoff zur Induzierung der Osmose und gegebenenfalls weitere pharmazeutisch annehmbare Hilfsstoffe und
c) einer Passage durch die Hülle a) für den Transport der im Kern enthaltenen Bestandteile in die umgebende wässrige Körperflüssigkeit.
2. Perorales therapeutisches System gemäss Anspruch 1 bestehend aus
a) einer Hülle aus für Wasser durchlässiger und für die Komponenten des wirkstoffhaltigen Kerns sowie für die in Magen- oder Darmsaft enthaltenen Ionen undurchlässiger, acylierter Cellulose,
b) einem Kern enthaltend den in Wasser schwerlöslichen Wirkstoff ausgenommen Carbamazepin oder eine Wirkstoffkombination, das 1:1-Gemisch (Gew.) von Vinylpyrrolidon-Vinylacetat-Copolymerisat mit Ethylenoxidhomopolymerisat, Natrium- oder Kaliumchlorid zur Induzierung der Osmose sowie gegebenenfalls weitere pharmazeutisch annehmbare Hilfsstoffe und
c) einer Passage durch die Hülle a) für den Transport der im Kern enthaltenen Bestandteile in die umgebende wässrige Körperflüssigkeit.
3. Perorales therapeutisches System gemäss Anspruch 1 bestehend aus
a) einer Hülle aus für Wasser durchlässiger und für die Komponenten des wirkstoffhaltigen Kerns sowie für die in Magen- oder Darmsaft, enthaltenen Ionen undurchlässiger Celluloseacetat,
b) einem Kern enthaltend den in Wasser schwerlöslichen Wirkstoff ausgenommen Carbamazepin oder eine Wirkstoffkombination, das 1:1-Gemisch (Gew.) aus Vinylpyrrolidon-Vinylacetat-Copolymerisat mit einem Molekulargewicht von 60000 ± 15000 und einem Monomerenverhältnis von ca. 60:40 (Gew.-%) und Ethylenoxidhomopolymerisat mit einem Polymerisationsgrad von 2000 bis 100000 und
c) einer Passage durch die Hülle a) für den Transport der im Kern enthaltenen Bestandteile in die umgebende wässrige Körperflüssigkeit.
4. perorales therapeutisches System gemäss einem der Ansprüche 1-3 in Tablettenform.
5. Verfahren zur Herstellung eines therapeutischen Systems gemäss Anspruch 1, dadurch gekennzeichnet, dass man die Bestandteile des Kerns miteinander vermischt, den Kern mit der Hülle überzieht und in der Hülle eine Oeffnung anbringt.
6. Therapeutisches System gemäss Anspruch 1 zur Anwendung bei der Behandlung des menschlichen oder tierischen Körpers.

**Patentansprüche Für folgenden Vertragsstaaten: GR, ES**

1. Verfahren zur Herstellung eines peroralen therapeutischen Systems für die Darreichung von in Wasser schwerlöslichen Wirkstoffen ausgenommen Carbamazepin bestehend aus
a) einer Hülle aus einem für Wasser durchlässigen und für die Komponenten des wirkstoffhaltigen Kerns undurchlässigen Material,
b) einem Kern enthaltend einen in Wasser schwerlöslichen Wirkstoff oder eine Wirkstoffkombination, ein hydrophiles, polymeres Quellmittel bestehend aus einer Mischung des Vinylpyrrolidon-Vinylacetat-Copolymerisats mit Ethylenoxidhomopolymerisat, gegebenenfalls einen wasserlöslichen Stoff zur Induzierung der Osmose und gegebenenfalls weitere pharmazeutisch annehmbare Hilfsstoffe und
c) einer Passage durch die Hülle a) für den Transport der im Kern enthaltenen Bestandteile in die umgebende wässrige Körperflüssigkeit, dadurch gekennzeichnet, dass man die Bestandteile des Kerns miteinander vermischt, den Kern mit der Hülle überzieht und in der Hülle eine Oeffnung anbringt.
2. Verfahren zur Herstellung eines peroralen therapeutischen Systems gemäss Anspruch 1 bestehend aus
a) einer Hülle aus für Wasser durchlässiger und für die Komponenten des wirkstoffhaltigen Kerns sowie für die in Magen- oder Darmsaft enthaltenen Ionen undurchlässiger, acylierter Cellulose, z.B. Celluloseacetat,

b) einem Kern enthaltend einen in Wasser schwerlöslichen Wirkstoff oder eine Wirkstoffkombination, das 1:1-Gemisch (Gew.) von Vinylpyrrolidon-Vinylacetat-Copolymerisat mit Ethylenoxidhomopolymerisat, Natrium- oder Kaliumchlorid zur Induzierung der Osmose sowie gegebenenfalls weitere pharmazeutisch annehmbare Hilfsstoffe und

c) einer Passage durch die Hülle a) für den Transport der im Kern enthaltenen Bestandteile in die umgebende wässrige Körperflüssigkeit, dadurch gekennzeichnet, dass man die in Anspruch 1 genannten Massnahmen durchführt.

3. Verfahren zur Herstellung eines peroralen therapeutischen Systems gemäss Anspruch 1 bestehend aus

a) einer Hülle aus für Wasser durchlässiger und für die Komponenten des wirkstoffhaltigen Kerns sowie für die in Magen- oder Darmsaft enthaltenen Ionen undurchlässigem Celluloseacetat,

b) einem Kern enthaltend einen in Wasser schwerlöslichen Wirkstoff ausgenommen Carbamazepin oder eine Wirkstoffkombination, das 1:1-Gemisch (Gew.) aus Vinylpyrrolidon-Vinylacetat-Copolymerisat mit einem Molekulargewicht von $60000 \pm 15000$ und einem Monomerenverhältnis von ca. 60:40 (Gew.-%) und Ethylenoxidhomopolymerisat mit einem Polymerisationsgrad von 2000 bis 100000 und

c) einer Passage durch die Hülle a) für den Transport der im Kern enthaltenen Bestandteile in die umgebende wässrige Körperflüssigkeit, dadurch gekennzeichnet, dass man die in Anspruch 1 genannten Massnahmen durchführt.

4. Verfahren zur Herstellung eines peroralen therapeutischen Systems gemäss einem der Ansprüche 1-3 in Tablettenform.


## Claims

**Claims for the following Contracting States : AT, BE, CH, DE, FR, GB, IT, LI, LU, NL, SE**

1. Peroral therapeutic system for the administration of active ingredients that are sparingly soluble in water, except carbamazepine, consisting of

a) a casing made of a material that is permeable to water and impermeable to the components of the core containing the active ingredient,

b) a core containing the active ingredient that is sparingly soluble in water or a mixture of such active ingredients, a hydrophilic, polymeric swelling agent consisting of a mixture of a vinylpyrrolidone/vinyl acetate copolymer with an ethylene oxide homopolymer, optionally a water-soluble substance for inducing osmosis and optionally other pharmaceutically acceptable adjuncts and

c) a passage through the casing a) for the transport of the constituents contained in the core into the surrounding aqueous body fluid.

2. Peroral therapeutic system according to claim 1, consisting of

a) a casing made of acylated cellulose that is permeable to water and impermeable to the components of the core containing the active ingredient and to the ions contained in gastric or intestinal juices,

b) a core containing the active ingredient that is sparingly soluble in water, except carbamazepine, or a mixture of such active ingredients, a 1:1 mixture (by weight) of a vinylpyrrolidone/vinyl acetate copolymer with an ethylene oxide homopolymer, sodium or potassium chloride for inducing osmosis and optionally other pharmaceutically acceptable adjuncts and

c) a passage through the casing a) for the transport of the constituents contained in the core into the surrounding aqueous body fluid.

3. Peroral therapeutic system according to claim 1, consisting of

a) a casing made of cellulose acetate that is permeable to water and impermeable to the components of the core containing the active ingredient and to the ions contained in gastric or intestinal juices,

b) a core containing the active ingredient that is sparingly soluble in water, except carbamazepine, or a mixture of such active ingredients, a 1:1 mixture (by weight) of a vinylpyrrolidone/vinyl acetate copolymer having a molecular weight of $60\,000 \pm 15\,000$ and a monomer ratio of approximately 60:40 (% by weight) and an ethylene oxide homopolymer having a degree of polymerisation of from 2000 to 100 000 and

c) a passage through the casing a) for the transport of the constituents contained in the core into the surrounding aqueous body fluid.

4. Peroral therapeutic system according to any one of claims 1 - 3 in tablet form.

5. Process for the manufacture of a therapeutic system according to claim 1, characterised in that the constituents of the core are mixed with one another, the core is covered with the casing and an opening is made

EP 0 277 092 B1

in the casing.

6. Therapeutic system according to claim 1 for use in the treatment of the human or animal body.

**Claims for the following Contracting States: GR, ES**

1. Process for the manufacture of a peroral therapeutic system for the administration of active ingredients that are sparingly soluble in water, except carbamazepine, consisting of

a) a casing made of a material that is permeable to water and impermeable to the components of the core containing the active ingredient,

b) a core containing an active ingredient that is sparingly soluble in water or a mixture of such active ingredients, a hydrophilic, polymeric swelling agent consisting of a mixture of a vinylpyrrolidone/vinyl acetate copolymer with an ethylene oxide homopolymer, optionally a water-soluble substance for inducing osmosis and optionally other pharmaceutically acceptable adjuncts and

c) a passage through the casing a) for the transport of the constituents contained in the core into the surrounding aqueous body fluid, characterised in that the constituents of the core are mixed with one another, the core is covered with the casing and an opening is made in the casing.

2. Process for the manufacture of a peroral therapeutic system according to claim 1, consisting of

a) a casing made of acylated cellulose, for example cellulose acetate, that is permeable to water and impermeable to the components of the core containing the active ingredient and to the ions contained in gastric or intestinal juices,

b) a core containing an active ingredient that is sparingly soluble in water or a mixture of such active ingredients, a 1:1 mixture (by weight) of a vinylpyrrolidone/vinyl acetate copolymer with an ethylene oxide homopolymer, sodium or potassium chloride for inducing osmosis and optionally other pharmaceutically acceptable adjuncts and

c) a passage through the casing a) for the transport of the constituents contained in the core into the surrounding aqueous body fluid, characterised in that the steps mentioned in claim 1 are carried out.

3. Process for the manufacture of a peroral therapeutic system according to claim 1, consisting of

a) a casing made of cellulose acetate that is permeable to water and impermeable to the components of the core containing the active ingredient and to the ions contained in gastric or intestinal juices,

b) a core containing an active ingredient that is sparingly soluble in water, except carbamazepine, or a mixture of such active ingredients, a 1:1 mixture (by weight) of a vinylpyrrolidone/vinyl acetate copolymer having a molecular weight of 60 000 ± 15 000 and a monomer ratio of approximately 60:40 (% by weight) and an ethylene oxide homopolymer having a degree of polymerisation of from 2000 to 100 000 and

c) a passage through the casing a) for the transport of the constituents contained in the core into the surrounding aqueous body fluid, characterised in that the steps mentioned in claim 1 are carried out.

4. Process for the manufacture of a peroral therapeutic system according to any one of claims 1 - 3 in tablet form.

**Revendications**

**Revendications pour les Etats contractants suivants : AT, BE, CH, DE, FR, GB, IT, LI, LU, NL, SE**

1. Système thérapeutique peroral pour l'administration de substances actives peu solubles dans l'eau à l'exception de la carbamazepine, constitué de :

a) une enveloppe de matière perméable à l'eau et imperméable aux composants du noyau contenant la substance active,

b) un noyau contenant la substance active ou une combinaison de substances actives peu solubles dans l'eau, un agent de gonflement polymère hydrophyle constitué d'un mélange de copolymères vinylpyrrolidone-acétate de vinyle avec un homopolymère d'oxyde d'éthylène, éventuellement un corps soluble dans l'eau pour induire une osmose et éventuellement d'autres additifs pharmaceutiquement acceptables, et

c) un passage à travers l'enveloppe a) pour le transport des composants contenus dans le noyau dans le liquide corporel aqueux environnant.

2. Système thérapeutique peroral selon la revendication 1, constitué de :

a) une enveloppe de cellulose acylée perméable à l'eau et imperméable aux composants du noyau contenant la substance active ainsi qu'aux ions contenus dans le suc gastrique ou intestinal,

b) un noyau contenant la substance active peu soluble dans l'eau à l'exception de la carbamazepine ou une combinaison de substances actives, le mélange 1:1 (en poids) de copolymères vinylpyrrolidone-acétate de vinyle avec un homopolymère d'oxyde d'éthylène, du chlorure de sodium ou de potassium pour induire une osmose ainsi qu'éventuellement d'autres additifs pharmaceutiquement acceptables, et

c) un passage à travers l'enveloppe a) pour le transport des composants contenus dans le noyau dans le liquide corporel aqueux environnant.

3. Système thérapeutique peroral selon la revendication 1, constitué de :

a) une enveloppe d'acétate de cellulose perméable à l'eau et imperméable aux composants du noyau contenant la substance active ainsi qu'aux ions contenus dans le suc gastrique ou intestinal,

b) un noyau contenant la substance active peu soluble dans l'eau à l'exception de la carbamazepine ou une combinaison de substances actives, le mélange 1:1 (en poids) de copolymères vinylpyrrolidone-acétate de vinyle ayant un poids moléculaire de 60 000 ± 15 000 et un rapport de monomères d'environ 60:40 (% en poids) et un homopolymère d'oxyde d'éthylène ayant un degré de polymérisation de 2000 à 100 000, et

c) un passage à travers l'enveloppe a) pour le transport des composants contenus dans le noyau dans le liquide corporel aqueux environnant.

4. Système thérapeutique peroral selon l'une des revendications 1-3, sous forme de comprimés.

5. Procédé de préparation d'un système thérapeutique selon la revendication 1, caractérisé en ce qu'on mélange les composants du noyau entre eux, en ce qu'on recouvre le noyau avec l'enveloppe et en ce qu'on produit une ouverture dans l'enveloppe.

6. Système thérapeutique selon la revendication 1, aux fins d'applications dans le traitement du corps humain ou animal.

**Revendications pour les Etats contractants suivants: GR, ES**

1. Procédé de préparation d'un système thérapeutique peroral pour l'administration de substances actives peu solubles dans l'eau à l'exception de la carbamazepine, constitué de :

a) une enveloppe faite d'une matière perméable à l'eau et imperméable aux composants du noyau contenant la substance active,

b) un noyau contenant une substance active peu soluble dans l'eau ou une combinaison de substances actives, un agent de gonflement polymère hydrophyle constitué d'un mélange de copolymères vinylpyrrolidone-acétate de vinyle avec un homopolymère d'oxyde d'éthylène, éventuellement un corps soluble dans l'eau pour induire une osmose et éventuellement d'autres additifs pharmaceutiquement acceptables, et

c) un passage à travers l'enveloppe a) pour le transport des composants contenus dans le noyau dans le liquide corporel aqueux environnant, caractérisé en ce qu'on mélange les composants du noyau entre eux, en ce qu'on recouvre le noyau avec l'enveloppe et en ce qu'on fait une ouverture dans l'enveloppe.

2. Procédé de préparation d'un système thérapeutique peroral selon la revendication 1, constitué de :

a) une enveloppe de cellulose acylée perméable à l'eau et imperméable aux composants du noyau contenant la substance active ainsi qu'aux ions contenus dans le suc gastrique ou intestinal, par exemple l'acétate de cellulose,

b) un noyau contenant une substance active ou une combinaison de substances actives peu solubles dans l'eau, le mélange 1:1 (en poids) de copolymères vinylpyrrolidone-acétate de vinyle avec un homopolymère d'oxyde d'éthylène, du chlorure de sodium ou de potassium pour induire l'osmose ainsi qu'éventuellement d'autres additifs pharmaceutiquement acceptables, et

c) un passage à travers l'enveloppe a) pour le transport des composants contenus dans le noyau dans le liquide corporel aqueux environnant, caractérisé en ce qu'on prend les mesures mentionnées dans la revendication 1.

3. Procédé de préparation d'un système thérapeutique peroral selon la revendication 1, constitué de :

a) une enveloppe d'acétate de cellulose perméable à l'eau et imperméable aux composants du noyau contenant la substance active ainsi qu'aux ions contenus dans le suc gastrique ou intestinal,

b) un noyau contenant une substance active peu soluble dans l'eau à l'exception de la carbamazepine ou une combinaison de substances actives, le mélange 1:1 (en poids) de copolymères vinylpyrrolidone-acétate de vinyle ayant un poids moléculaire de 60 000 ± 15 000 et un rapport de monomères d'environ 60:40 (% en poids) et un homopolymère d'oxyde d'éthylène ayant un degré de polymérisation de 2000 à 100 000, et

c) un passage à travers l'enveloppe a) pour le transport des composants contenus dans le noyau dans le liquide corporel aqueux environnant, caractérisé en ce qu'on prend les mesures mentionnées dans la revendication 1.

4. Procédé de préparation d'un système thérapeutique peroral selon l'une des revendications 1-3, sous forme de comprimés.